# EUROPÄISCHE PATENTSCHRIFT

(11) **EP 1 462 157 B1**
(45) Veröffentlichungstag und Bekanntmachung des Hinweises auf die Patenterteilung: **14.01.2009**
(21) Anmeldenummer: 03006538.7
(22) Anmeldetag: 24.03.2003
(51) Int. Cl.: B01J 13/02

(54) **Mikrokapseln (XXIII)**
Microcapsules (XXIII)
Microcapsules (XXIII)

(43) Veröffentlichungstag der Anmeldung: 29.09.2004
(73) Patentinhaber: Cognis IP Management GmbH, 40589 Düsseldorf (DE)
(72) Erfinder: Viladot Petit, Josep-Lluis, Dr., 08018 Barcelona (ES); Asensio, Juan-Antonio, 08820 El Prat de Llobregat (Barcelona) (ES)

(56) Entgegenhaltungen:
- EP-A- 1 284 127
- GB-A- 1 483 542

## Beschreibung

### Gebiet der Erfindung

Die Erfindung befindet sich auf dem Gebiet der Verkapselung von Wirkstoffen und betrifft neue Mikrokapseln, ein Verfahren zur ihrer Herstellung sowie ihre Verwendung im Bereich der Kosmetik, Pharmazie und Lebensmittelzusatzstoffe.

### Stand der Technik

Unter dem Begriff "Mikrokapsel" werden sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden flüssige Wirkstoffe in einer Matrix aufgenommen ("microsponge"), die als Mikropartikel zusätzlich mit filmbildenden Polymeren umhüllt sein können. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natriumoder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinghydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.
Beispiele für Mikrokapseln des Stands der Technik sind folgende Handelsprodukte (in Klammein angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide). Chitosanmikrokapseln und Verfahren zu ihrer Herstellung sind Gegenstand früherer Patenanmeldungen der Patentanmelderin [WO 01/01926, WO 01/01927, WO 01/01928, WO 01/01929].

Die Freisetzung der Wirkstoffe aus den Mikrokapseln erfolgt üblicherweise während der Anwendung der sie enthaltenden Zubereitungen durch Zerstörung der Hülle infolge mechanischer, thermischer, chemischer oder enzymatischer Einwirkung. Von Nachteil ist dabei, dass die Mikrokapseln die kontrollierte Freisetzung der Wirkstoffe aus ihrem Innern nicht oder nur in unzureichendem Maße zulassen und die Kapseln eine ungenügende Stabilität in Gegenwart von Tensiden, zumal anionischen Tensiden aufweisen. Die Aufgabe der vorliegenden Erfindung hat somit darin bestanden, gerade diese Nachteile zu überwinden.

### Beschreibung der Erfindung

Gegenstand der Erfindung sind neue Mikrokapseln mit mittleren Durchmessern im Bereich von 0,0001 bis 5, vorzugsweise 0,001 bis 1 und insbesondere 0,01 bis 0,5 mm, bestehend aus einer Hüllmembran und einer die Wirkstoffe enthaltenden Matrix, die dadurch erhältlich sind, dass man
(a1) aus Gelbildnern, anionischen Polymeren, Polyalkylenglykolen und Wirkstoffen eine Matrix zubereitet,
(a2) gegebenenfalls die Matrix in einer Ölphase dispergiert,
(a3) die dispergierte Matrix mit wässrigen Lösungen synthetischer Kationpolymere behandelt und gegebenenfalls dabei die Ölphase entfernt.
oder
(b1) aus Gelbildnern, synthetischen kationischen Polymeren, Polyalkylenglykolen und Wirkstoffen eine Matrix zubereitet,
(b2) gegebenenfalls die Matrix in einer Ölphase dispergiert,
(b3) die dispergierte Matrix mit wässrigen Lösungen anionischer Polymere behandelt und gegebenenfalls dabei die Ölphase entfernt.

Die neuen Mikrokapseln entstehen dabei durch Koazervation zwischen den anionischen und kationischen Polymeren, welche an den lipophilen Grenzflächen der O/W-Emulsionströpfchen stattfindet. Auf diese Weise gelingt es, auch solche Stoffe zu verkapseln, bei denen dies üblicherweise sehr schwierig ist. Zudem zeichnen sich die Mikrokapseln infolge des Einbaus von Polyethylenglykolen durch eine deutlich verbesserte Tensidstabilität aus. In einer bevorzugten Ausführungsform der Erfindung sind die Mikrokapseln zudem frei von natürlichen Kationpolymeren, insbesondere von Chitinen und Chitosanen, da diese für den Einsatz im Nahrungsmittelbereich derzeit nur beschränkt zugelassen sind.

Ein weiterer Gegenstand der Erfindung betrifft ein Verfahren zur Herstellung von Mikrokapseln mit mittleren Durchmessern im Bereich von 0,0001 bis 5 mm, bestehend aus einer Hüllmembran und einer die Wirkstoffe enthaltenden Matrix, bei dem man
(a1) aus Gelbildnern, anionischen Polymeren, Polyalkylenglykolen und Wirkstoffen eine Matrix zubereitet,
(a2) gegebenenfalls die Matrix in einer Ölphase dispergiert,
(a3) die dispergierte Matrix mit wässrigen Lösungen synthetischer Kationpolymere behandelt und gegebenenfalls dabei die Ölphase entfernt.
oder
(b1) aus Gelbildnern, synthetischen kationischen Polymeren, Polyalkylenglykolen und Wirkstoffen eine Matrix zubereitet,
(b2) gegebenenfalls die Matrix in einer Ölphase dispergiert,
(b3) die dispergierte Matrix mit wässrigen Lösungen anionischer Polymere behandelt und gegebenenfalls dabei die Ölphase entfernt.

### Gelbildner

Im Sinne der Erfindung werden als Gelbildner vorzugsweise solche Stoffe in Betracht gezogen, welche die Eigenschaft zeigen, in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3-und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### Anionpolymere

Als anionische Polymere eignen sich neben anionischen Polysacchariden, wie z.B. Carboxymethylcellulose, oder Poly(meth)acrylsäuren und deren Derivaten, wie z.B. Salzen und Estern, vorzugsweise Salze der Alginsäure. Bei der Alginsäure handelt es sich um ein Gemisch carboxylgruppenhaltiger Polysaccharide mit folgendem idealisierten Monomerbaustein:

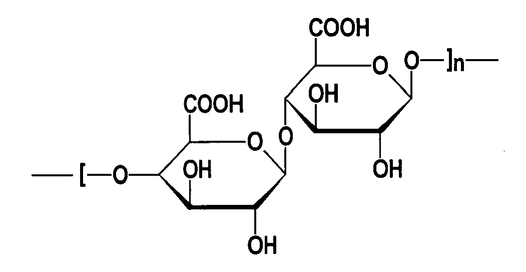

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Die Einsatzmenge der Anionpolymere beträgt in der Regel - bezogen auf die Mikrokapseln - 0,01 bis 1, vorzugsweise 0,05 bis 0,1 Gew.-%.

### Polyalkylenglykole

Der Zusatz der Polyalkylenglykole führt zu einer Stabilisierung der Kapseln und verbessert insbesondere ihre Resistenz gegen den Einfluss von Tensiden. Bei diesen Polymeren kann es sich um Oligomere des Ethylenoxids oder Propylenoxids bzw. um Gemische dieser beiden - in Random- wie in Blockverteilung - handeln. Üblicherweise kommen solche Polyalkylenglykole, vorzugsweise aber Polyethylenglykole zum Einsatz, die ein mittleres Molekulargewicht von 100 bis 5.000, vorzugsweise von 200 bis 2.000 aufweisen.

### Wirkstoffe

Die Auswahl der zu verkapselnden Wirkstoffe ist an sich unkritisch. Es sollte sich um wasseroder öllösliche Substanzen handeln, welche beispielsweise ausgewählt sein können aus der Gruppe der Fette und Wachse, Perlglanzwachse, Lecithine, Phospholipide, biogene Wirkstoffe, Enzyme, UV-Lichtschutzfaktoren, Antioxidantien, Deodorantien, Antitranspirantien, Antischuppenmittel, Filmbildner, Insektenrepellentien, Selbstbräuner, Tyrosininhibitoren, Parfümöle, Farbstoffe und dergleichen.

### • Fette und Wachse

Typische Beispiele für Fette und Wachse sind Glyceride, d.h. feste oder flüssige pflanzliche oder tierische Produkte, die im wesentlichen aus gemischten Glycerinestem höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### • Enzyme

Als zu verkapselnde Enzyme kommen insbesondere solche aus der Klasse der Hydrolasen, wie der Proteasen, Esterasen, Lipasen bzw. lipolytisch wirkenden Enzyme, Amylasen, Cellulasen bzw. andere Glykosylhydrolasen und Gemische der genannten Enzyme in Frage. Besonders gut geeignet sind aus Bakterienstämmen oder Pilzen, wie Bacillus subtilis, Bacillus licheniformis, Streptomyces griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease- und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt.

### • UV-Lichtschutzfaktoren und Lichtschutzpigmente

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen: 3-Benzylidencampher bzw. 3-Benzylidennorcampher und dessen Derivate, z.B. 3-(4-Methylbenzyliden)campher; 4-Aminobenzoe-säurederivate, vorzugsweise 4-(Dimethylamino)benzoesäure-2-ethyl-hexylester, 4-(Dimethylamino)benzoesäure-2-octylester und 4-(Dimethylamino)benzoe-säureamyl-ester; Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenyl-zimtsäure-2-ethylhexylester (Octocrylene); Ester der Salicylsäure, vorzugsweise Salicylsäure-2-ethylhexylester, Salicylsäure-4-iso-propylbenzylester, Salicylsäurehomomenthylester; Derivate des Benzophenons, vorzugsweise 2-Hydroxy-4-methoxybenzo-phenon, 2-Hydroxy-4-methoxy-4'-methylbenzophenon, 2,2'-Dihydroxy-4-methoxyben-zophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2`-ethyl-1`-hexyl-oxy)-1,3,5-triazin und Octyl Triazonoder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2. 1.0)decan-De-rivate.

Als wasserlösliche Substanzen kommen in Frage: 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzo-phenon-5-sulfonsäure und ihre Salze; Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol® 1789) und 2-Cyano-3,3-phenylzimtsäure-2-ethylhexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

### • Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Typische Beispiele für geeignete Pflanzenextrakte sind die Wirkstoffe folgender Pflanzen : *Aesculus hippocastanum* (Rosskastanie), *Argania spinosa, Babtista tinctoria* (wilder Indigo), *Cantella asiatica, Camelilla sinensis* (Grüner Tee), *Chamonella recutita* (Kamille), *Ginkgo biloba* (Ginkgo), *Oleo europea* (Olive), *Litschi chinensis* (Litchi), *Melissa officinalis* (Zitronenmelisse), *Panax ginseng* (Ginseng), *Passiflora incarnata* (Passionsblume), *Prunus dulcis* (Süßmandel), *Pterocarpus marsupium, Ruscus aculeatus, Trifolium pratense* (Red Clover), *Uva ursi* (Bärtraube), *Vaccinium myrtillus* (Blaubeere), *Vigna acontifolia,* und *Vitis vinifera* (wilder Wein).

Typische Beispiele für geeignete Antioxidantien sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### • Deodorantien, keimhemmende Mittel und Enzyminhibitoren

Als adstringierende Deodorantien eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkoniumpentachlorohydrat und deren Komplexverbindungen z. B. mit Aminosäuren wie Glycin.

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarb-anilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder - phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

### • Weitere Wirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climba-zole), Ketoconazol®, (4-Acetyl-1-{-4-[2-(2.4-dichlorphenyl) r-2-(1H-imidazol-1-ylme-thyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachloro-phen), Undexylensäure Monoethanolamid Sulfosuccinat Na-Salz, Lamepon® UD (Protein-Undecylensäurekondensat), Zinkpyrithion, Aluminiumpyrithion und Magne-siumpyrithion / Dipyrithion-Magnesiumsulfat in Frage.

Als Insekten-Repellentien kommen N,N-Diethyl-m-toluamid, 1,2-Pentandiol oder Ethyl Butylacetylaminopropionate in Frage Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### • Parfümöle

Als Parfümöle, die verkapselt werden können seien genannt : Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Alle genannten Wirkstoffe werden - bezogen auf die Mikrokapseln- üblicherweise in Konzentrationen von 0,001 bis 10, vorzugsweise 0,5 bis 5 und insbesondere 1 bis 2 Gew.-% eingesetzt.

### Ölphase

In einer besonderen Ausführungsform der Erfindung kann die im ersten Schritt hergestellte Matrix vor der Verkapselung in einer Ölphase dispergiert werden. Für diesen Zwecke kommen beispielsweise in Frage : Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane. Die Menge der Ölkörper kann bezogen auf die Mikrokapseln 10 bis 30 und vorzugsweise 15 bis 30 Gew.-% betragen.

### Emulgatoren

Für den Fall, dass die Matrix vor der Verkapselung in einer Ölphase dispergiert wird, empfiehlt es sich, Emulgatoren mitzuverwenden, um eine homogene Dispersion herzustellen. Als Emulgatoren kommen grundsätzlich anionische, kationische oder ampholytische Tenside in Frage. Vorzugsweise werden jedoch nichtionogene Tenside eingesetzt, welche beispielsweise aus mindestens einer der folgenden Gruppen ausgewählt sein können.
- Anlagerungsprodukte von 2 bis 30 Mol Ethylenoxid und/ oder 0 bis 5 Mol Propylenoxid an lineare Fettalkohole mit 8 bis 22 Kohlenstoffatomen, an Fettsäuren mit 12 bis 22 Kohlenstoffatomen, an Alkylphenole mit 8 bis 15 Kohlenstoffatomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich;
- Polyalkylenglycole sowie
- Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Die Konzentration der Emulgatoren kann bezogen auf die Mikrokapseln 1 bis 10 und vorzugsweise 2 bis 8 Gew.-% betragen.

### Synthetische Kationpolymere

Unter dem Begriff "synthetische Kationpolymere" sind diejenigen kationaktiven Polymere zu verstehen, die auf synthetischem Wege erhalten werden, d.h. zu deren Herstellung nicht auf natürliche polymere Ausgangsstoffe, insbesondere nicht auf Chitin oder Chitosan zurückgegriffen werden muss; die erfindungsgemäßen Mikrokapseln sind vielmehr frei von diesen Stoffen. Typische Beispiele sind beispielsweise Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, Kondensationsprodukte aus Dihalogenalkylen, wie z.B. Dibrombutan mit Bisdialkylaminen, wie z.B. Bis-Dimethylamino-1,3-propan, sowie quatemierte Ammoniumsalz-Polymere, wie z.B. Mirapol® A-15, Mirapol® AD-1, Mirapol® AZ-1 der Firma Miranol. Die Einsatzmenge der Kationpolymere liegt - bezogen auf die Mikrokapseln - vorzugsweise im Bereich von 0,01 bis 1, vorzugsweise 0,05 bis 0,1 Gew.-%.

### Herstellung der Mikrokapseln

Zur Herstellung der erfindungsgemäßen Mikrokapseln wird zunächst eine wässrige Matrix hergestellt, die neben den Gelbildnem und den anionischen oder kationischen Polymeren auch die Polyethylenglykole sowie die Wirkstoffe enthalten. Falls gewünscht, kann die Matrix in einer Ölphase dispergiert werden; in diesem Fall empfiehlt sich die Mitverwendung geeigneter Emulgatoren, um eine homogene Verteilung sicherzustellen. Die Dispergierung in der Ölphase ist dann von Vorteil, wenn besonders kleine Mikrokapseln gewünscht werden. Die Membranbildung erfolgt durch Zugabe der entgegengesetzt geladenen Polymerlösung. Wenn also die Matrix bereits das Anionpolymer enthält, wird als entgegengesetzt geladene Komponente ein Kationpolymer benötigt und umgekehrt. Das Inkontaktbringen kann durch Einrühren vorzugsweise aber durch Eintropfen erfolgen. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diesten von Fettsäuren, Polyacrylaten, Polyacrylamiden und dergleichen noch unterstützt werden kann. Abschließend können die Mikrokapseln von der wässrigen Phase beispielsweise durch Dekantieren, Filtrieren oder Zentrifugieren abgetrennt werden.

### Gewerbliche Anwendbarkeit

Ein weiterer Gegenstand der Erfindung betrifft die Verwendung der erfindungsgemäßen Mikrokapseln zur Herstellung von kosmetischen und/oder pharmazeutischen Zubereitungen, wie beispielsweise Haarshampoos, Haarlotionen, Schaumbäder, Duschbäder, Cremes, Gele, Lotionen, alkoholische und wässrig/alkoholische Lösungen, Emulsionen, Wachs/ Fett-Massen, Stiftpräparaten, Pudern oder Salben. Diese können ihrerseits wieder Wirkstoffe, Ölkörper und Emulgatoren in unverkapselter Form enthalten, wie sie bereits eingangs beschrieben worden sind; auf eine erneute Aufzählung wird daher verzichtet. Bei den Zubereitungen kann es sich auch um Lebensmittelzusatzstoffe handeln, beispielsweise um Additive für Sportlernahrung und dergleichen. Die Mikrokapseln können in diesen Mitteln in Mengen von 1 bis 50, vorzugsweise 5 bis 15 Gew.-% enthalten sein.

### Beispiele

### Beispiel 1

In einem 250-ml-Dreihalskolben mit Rührer und Rückflusskühler wurden 0,7 g Agar-Agar in 36,3 ml Wasser in der Siedehitze aufgelöst und anschließend mit 25 g einer 2 Gew.-%igen wässrigen Lösung von Natriumalginat versetzt. Danach wurden 15 g Polyethylenglykol (Molgewicht 200), 0,5 g Phenonip® (Konservierungsmittelmischung), 10 g Glycerin, 2,5 g einer Pigmentmischung bestehend aus Mica und Eisenoxiden sowie 10 g Mineralöl, 0,16 g Tocopherolacetat und 0,01 g Carotinpalmitat unter intensivem Rühren hinzugegeben. Zur Verkapselung wurde die Matrix in eine wässrige 1 Gew.-%ige Kationpolymerlösung (Polyquart® 701/N) getropft, die zusätzlich noch 0,5 Gew.-% Calciumchlorid enthielt. Die resultierenden Mikrokapseln wiesen einen mittleren Durchmesser von 1 mm auf.

### Beispiel 2

In einem 250-ml-Dreihalskolben mit Rührer und Rückflusskühler wurden 0,7 g Agar-Agar in 36,3 ml Wasser in der Siedehitze aufgelöst und anschließend mit 25 g einer 2 Gew.-%igen wässrigen Lösung von Natriumalginat versetzt. Danach wurden 15 g Polyethylenglykol (Molgewicht 200 , "PEG-200"), 0,5 g Phenonip® (Konservierungsmittelmischung), 10 g Glycerin, 2,5 g einer Pigmentmischung bestehend aus Mica und Eisenoxiden sowie 10 g Mineralöl, 0,16 g Tocopherolacetat und 0,01 g Carotinpalmitat unter intensivem Rühren hinzugegeben. Zur Verkapselung wurde die Matrix in 100 ml Mineralöl eingerührt, abgekühlt, filtriert, zunächst mit 200 g einer 1 Gew.-%igen Lösung von Polysorbat-20 und so dann mit einer gleichen Menge einer 1 Gew.-%igen Lösung von Polyquart® 701/N behandelt. Abschließend wurden die Mikrokapseln abgetrennt und mit konservierungsmittelhaltigem Wasser gewaschen. Die resultierenden Mikrokapseln wiesen einen mittleren Durchmesser von 1 mm auf.

### Beispiel 3

In einem 250-ml-Dreihalskolben mit Rührer und Rückflusskühler wurden 0,7 g Agar-Agar in 36,3 ml Wasser in der Siedehitze aufgelöst und anschließend mit 25 g einer 1 Gew.-%igen wässrigen Lösung von Polyquart® 701/N versetzt. Danach wurden 15 g Polyethylenglykol (Molgewicht 200), 0,5 g Phenonip® (Konservierungsmittelmischung), 10 g Glycerin, 2,5 g einer Pigmentmischung bestehend aus Mica und Eisenoxiden sowie 10 g Mineralöl, 0,16 g Tocopherolacetat und 0,01 g Carotinpalmitat unter intensivem Rühren hinzugegeben. Zur Verkapselung wurde die Matrix in eine wässrige 1 Gew.-%ige Lösung von Natriumalginat getropft, die zusätzlich noch 0,5 Gew.-% Calciumchlorid enthielt. Die resultierenden Mikrokapseln wiesen einen mittleren Durchmesser von 1 mm auf.

## Patentansprüche

1. Mikrokapseln mit mittleren Durchmessern im Bereich von 0,0001 bis 5 mm, bestehend aus einer Hüllmembran und einer die Wirkstoffe enthaltenden Matrix, **dadurch** erhältlich, dass man
(a1) aus Gelbildnern, anionischen Polymeren, Polyalkylenglykolen und Wirkstoffen eine Matrix zubereitet,
(a2) gegebenenfalls die Matrix in einer Ölphase dispergiert,
(a3) die dispergierte Matrix mit wässrigen Lösungen synthetischer Kationpolymere behandelt und gegebenenfalls dabei die Ölphase entfernt.
oder
(b1) aus Gelbildnern, synthetischen kationischen Polymeren, Polyalkylenglykolen und Wirkstoffen eine Matrix zubereitet,
(b2) gegebenenfalls die Matrix in einer Ölphase dispergiert,
(b3) die dispergierte Matrix mit wässrigen Lösungen anionischer Polymere behandelt und gegebenenfalls dabei die Ölphase entfernt.

2. Mikrokapseln nach Anspruch 1, **dadurch gekennzeichnet, dass** sie frei sind von natürlichen Kationpolymeren, insbesondere von Chitinen und Chitosanen.

3. Verfahren zur Herstellung von Mikrokapseln mit mittleren Durchmessern im Bereich von 0,0001 bis 5 mm, bestehend aus einer Hüllmembran und einer die Wirkstoffe enthaltenden Matrix, bei dem man
(a1) aus Gelbildnern, anionischen Polymeren, Polyalkylenglykolen und Wirkstoffen eine Matrix zubereitet,
(a2) gegebenenfalls die Matrix in einer Ölphase dispergiert,
(a3) die dispergierte Matrix mit wässrigen Lösungen synthetischer Kationpolymere behandelt und gegebenenfalls dabei die Ölphase entfernt.
oder
(b1) aus Gelbildnern, synthetischen kationischen Polymeren, Polyalkylenglykolen und Wirkstoffen eine Matrix zubereitet,
(b2) gegebenenfalls die Matrix in einer Ölphase dispergiert,
(b3) die dispergierte Matrix mit wässrigen Lösungen anionischer Polymere behandelt und gegebenenfalls dabei die Ölphase entfernt.

4. Verfahren nach Anspruch 3, **dadurch gekennzeichnet, dass** man Gelbildner einsetzt, die ausgewählt sind aus der Gruppe, die von Heteropolysacchariden und Proteinen gebildet wird.

5. Verfahren nach den Ansprüchen 3 und 4, **dadurch gekennzeichnet, dass** man anionische Polymere einsetzt, die ausgewählt sind aus der Gruppe, die gebildet wird von anionischen Polysacchariden, Poly(meth)acrylsäuren und deren Derivaten sowie Alginsäure und deren Salzen.

6. Verfahren nach mindestens einem der Ansprüche 3 bis 5, **dadurch gekennzeichnet, dass** man Polyalkylenglykole einsetzt, die ein mittleres Molekulargewicht von 100 bis 5.000 Dalton aufweisen.

7. Verfahren nach mindestens einem der Ansprüche 3 bis 6, **dadurch gekennzeichnet, dass** man Wirkstoffe einsetzt, die ausgewählt sind aus der Gruppe, die gebildet wird von Fetten und Wachsen, Perlglanzwachsen, Lecithinen, Phospholipiden, biogenen Wirkstoffen, Enzymen, UV-Lichtschutzfaktoren, Antioxidantien, Deodorantien, Antitranspirantien, Antischuppenmitteln, Filmbildnern, Insektenrepellentien, Selbstbräunern, Tyrosininhibitoren, Parfümölen und Farbstoffen.

8. Verfahren nach mindestens einem der Ansprüche 3 bis 7, **dadurch gekennzeichnet, dass** man die Matrix in einer Ölphase dispergiert, die gebildet wird von Guerbetalkoholen auf Basis von Fettalkoholen mit 6 bis 18 Kohlenstoffatomen; Estern von linearen C₆-C₂₂-Fettsäuren mit linearen oder verzweigten C₆-C₂₂-Fettalkoholen; Estern von verzweigten C₆-C₁₃-Carbonsäuren mit linearen oder verzweigten C₆-C₂₂-Fettalkoholen; Estern von linearen C₆-C₂₂-Fettsäuren mit verzweigten Alkoholen; Estern von C₁₈-C₃₈-Alkylhydroxycarbonsäuren mit linearen oder verzweigten C₆-C₂₂-Fettalkoholen; Estern von linearen und/oder verzweigten Fettsäuren mit mehrwertigen Alkoholen und/oder Guerbetalkoholen; Triglyceriden auf Basis C₆-C₁₀-Fettsäuren; flüssigen Mono-/Di-/Triglyceridmischungen auf Basis von C₆-C₁₈-Fettsäuren; Estern von C₆-C₂₂-Fettalkoholen und/oder Guerbetalkoholen mit aromatischen Carbonsäuren; Estern von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen; pflanzlichen Ölen; verzweigten primären Alkoholen; substituierten Cyclohexanen; linearen und verzweigte C₆-C₂₂-Fettalkoholcarbonaten; Guerbetcarbonaten auf Basis von Fettalkoholen mit 6 bis 18 Kohlenstoffatomen; Estern der Benzoesäure mit linearen und/oder verzweigten C₆-C₂₂-Alkoholen; linearen oder verzweigten, symmetrischen oder unsymmetrischen Dialkylethern mit 6 bis 22 Kohlenstoffatomen pro Alkylgruppe; Ringöffnungsprodukten von epoxidierten Fettsäureestern mit Polyolen; Siliconölen sowie aliphatischen bzw. naphthenischen Kohlenwasserstoffen und Mineralölen sowie deren Gemischen.

9. Verfahren nach mindestens einem der Ansprüche 3 bis 8, **dadurch gekennzeichnet, dass** man synthetische Kationpolymeren einsetzt, die ausgewählt sind aus der Gruppe, die gebildet wird von Copolymeren von Diallylammoniumsalzen und Acrylamiden, quaternierten Vinylpyrrolidon/Vinylimidazol-Polymeren, Kondensationsprodukten von Polyglycolen und Aminen, Polyethyleniminen, kationischen Siliconpolymeren, Copolymeren der Adipinsäure und Dimethylaminohydroxypropyldiethylentriamin, Copolymeren der Acrylsäure mit Dimethyldiallylammoniumchlorid, Polyaminopolyamiden sowie deren vernetzten wasserlöslichen Polymeren, Kondensationsprodukten aus Dihalogenalkylen und Bisdialkylaminen sowie quaternierten Ammoniumsalzpolymeren.

10. Verwendung von Mikrokapseln nach Anspruch 1 zur Herstellung von kosmetischen und/oder pharmazeutischen Mitteln oder Lebensmittelzubereitungen.

## Claims

1. Microcapsules with average diameters in the range from 0.0001 to 5 mm, consisting of a coating membrane and a matrix comprising the active ingredients, obtainable by
(a1) preparing a matrix from gel formers, anionic polymers, polyalkylene glycols and active ingredients,
(a2) optionally dispersing the matrix in an oil phase,
(a3) treating the dispersed matrix with aqueous solutions of synthetic cationic polymers and optionally removing the oil phase in the process,
or
(b1) preparing a matrix from gel formers, synthetic cationic polymers, polyalkylene glycols and active ingredients,
(b2) optionally dispersing the matrix in an oil phase,
(b3) treating the dispersed matrix with aqueous solutions of anionic polymers and optionally removing the oil phase in the process.

2. Microcapsules according to Claim 1, **characterized in that** they are free from natural cationic polymers, in particular from chitins and chitosans.

3. Process for the preparation of microcapsules with average diameters in the range from 0.0001 to 5 mm, consisting of a coating membrane and a matrix comprising the active ingredients, in which
(a1) a matrix is prepared from gel formers, anionic polymers, polyalkylene glycols and active ingredients,
(a2) optionally the matrix is dispersed in an oil phase,
(a3) the dispersed matrix is treated with aqueous solutions of synthetic cationic polymers and optionally the oil phase is removed in the process,
or
(b1) a matrix is prepared from gel formers, synthetic cationic polymers, polyalkylene glycols and active ingredients,
(b2) optionally the matrix is dispersed in an oil phase,
(b3) the dispersed matrix is treated with aqueous solutions of anionic polymers and optionally the oil phase is removed in the process.

4. Process according to Claim 3, **characterized in that** gel formers are used which are selected from the group formed by heteropolysaccharides and proteins.

5. Process according to Claims 3 and 4, **characterized in that** anionic polymers are used which are selected from the group formed by anionic polysaccharides, poly(meth)acrylic acids and derivatives thereof, and also alginic acid and salts thereof.

6. Process according to at least one of Claims 3 to 5, **characterized in that** polyalkylene glycols are used which have an average molecular weight of from 100 to 5000 daltons.

7. Process according to at least one of Claims 3 to 6, **characterized in that** active ingredients are used which are selected from the group formed by fats and waxes, pearlescent waxes, lecithins, phospholipids, biogenic active ingredients, enzymes, UV sun protection factors, antioxidants, deodorants, antiperspirants, antidandruff agents, film formers, insect repellents, self-tanning agents, tyrosine inhibitors, perfume oils and dyes.

8. Process according to at least one of Claims 3 to 7, **characterized in that** the matrix is dispersed in an oil phase which is formed by Guerbet alcohols based on fatty alcohols having 6 to 18 carbon atoms; esters of linear C₆-C₂₂-fatty acids with linear or branched C₆-C₂₂-fatty alcohols; esters of branched C₆-C₁₃-carboxylic acids with linear or branched C₆-C₂₂-fatty alcohols; esters of linear C₆-C₂₂-fatty acids with branched alcohols; esters of C₁₈-C₃₈-alkylhydroxycarboxylic acids with linear or branched C₆-C₂₂-fatty alcohols; esters of linear and/or branched fatty acids with polyhydric alcohols and/or Guerbet alcohols; triglycerides based on C₆-C₁₀-fatty acids; liquid mono-/di-/triglyceride mixtures based on C₆-C₁₈-fatty acids; esters of C₆-C₂₂-fatty alcohols and/or Guerbet alcohols with aromatic carboxylic acids; esters of C₂-C₁₂-dicarboxylic acids with linear or branched alcohols having 1 to 22 carbon atoms or polyols having 2 to 10 carbon atoms and 2 to 6 hydroxyl groups; vegetable oils; branched primary alcohols; substituted cyclohexanes; linear and branched C₆-C₂₂-fatty alcohol carbonates; Guerbet carbonates based on fatty alcohols having 6 to 18 carbon atoms; esters of benzoic acid with linear and/or branched C₆-C₂₂-alcohols; linear or branched, symmetrical or asymmetrical dialkyl ethers having 6 to 22 carbon atoms per alkyl group; ring-opening products of epoxidized fatty acid esters with polyols; silicone oils, and aliphatic or naphthenic hydrocarbons and mineral oils, and mixtures thereof.

9. Process according to at least one of Claims 3 to 8, **characterized in that** synthetic cationic polymers are used which are selected from the group formed by copolymers of diallylammonium salts and acrylamides, quaternized vinylpyrrolidone/vinylimidazole polymers, condensation products of polyglycols and amines, polyethyleneimines, cationic silicone polymers, copolymers of adipic acid and dimethylaminohydroxypropyldiethylenetriamine, copolymers of acrylic acid with dimethyldiallylammonium chloride, polyaminopolyamides, and crosslinked watersoluble polymers thereof, condensation products of dihaloalkyls and bisdialkylamines, and quaternized ammonium salt polymers.

10. Use of microcapsules according to Claim 1 for the preparation of cosmetic and/or pharmaceutical compositions or food preparations.

## Revendications

1. Microcapsules présentant des diamètres moyens dans la plage de 0,0001 à 5 mm, constituées par une membrane enveloppante et une matrice contenant les substances actives, pouvant être obtenues en ce que
(a1) on prépare, à partir de gélifiants, de polymères anioniques, de polyalkylèneglycols et de substances actives une matrice,
(a2) on disperse le cas échéant la matrice dans une phase huileuse,
(a3) on traite la matrice dispersée avec des solutions aqueuses de polymères cationiques synthétiques et on élimine le cas échéant la phase huileuse pendant ce traitement,
ou
(b1) on prépare, à partir de gélifiants, de polymères cationiques synthétiques, de polyalkylèneglycols et de substances actives une matrice,
(b2) on disperse le cas échéant la matrice dans une phase huileuse,
(b3) on traite la matrice dispersée avec des solutions aqueuses de polymères anioniques et on élimine le cas échéant la phase huileuse pendant ce traitement.

2. Microcapsules selon la revendication 1, **caractérisées en ce qu'**elles sont exemptes de polymères cationiques naturels, en particulier de chitines et de chitosanes.

3. Procédé pour la préparation de microcapsules présentant des diamètres moyens dans la plage de 0,0001 à 5 mm, constituées par une membrane enveloppante et une matrice contenant les substances actives, dans lequel
(a1) on prépare, à partir de gélifiants, de polymères anioniques, de polyalkylèneglycols et de substances actives une matrice,
(a2) on disperse le cas échéant la matrice dans une phase huileuse,
(a3) on traite la matrice dispersée avec des solutions aqueuses de polymères cationiques synthétiques et on élimine le cas échéant la phase huileuse pendant ce traitement,
ou
(b1) on prépare, à partir de gélifiants, de polymères cationiques synthétiques, de polyalkylèneglycols et de substances actives une matrice,
(b2) on disperse le cas échéant la matrice dans une phase huileuse,
(b3) on traite la matrice dispersée avec des solutions aqueuses de polymères anioniques et on élimine le cas échéant la phase huileuse pendant ce traitement.

4. Procédé selon la revendication 3, **caractérisé en ce qu'**on utilise des gélifiants qui sont choisis dans le groupe formé par les hétéropolysaccharides et les protéines.

5. Procédé selon les revendications 3 et 4, **caractérisé en ce qu'**on utilise des polymères anioniques qui sont choisis dans le groupe formé par les polysaccharides anioniques, les poly(acides (méth)acryliques) et leurs dérivés ainsi que l'acide alginique et ses sels.

6. Procédé selon au moins l'une quelconque des revendications 3 à 5, **caractérisé en ce qu'**on utilise des polyalkylèneglycols qui présentent un poids moléculaire moyen de 100 à 5000 Daltons.

7. Procédé selon au moins l'une quelconque des revendications 3 à 6, **caractérisé en ce qu'**on utilise des substances actives, qui sont choisies dans le groupe formé par les graisses et les cires, les cires nacrées, les lécithines, les phospholipides, les substances actives biogènes, les enzymes, les facteurs de protection contre la lumière UV, les antioxydants, les déodorants, les antiperspirants, les agents antipelliculaires, les agents filmogènes, les répulsifs d'insectes, les auto-bronzants, les inhibiteurs de tyrosine, les huiles parfumées et les colorants.

8. Procédé selon au moins l'une quelconque des revendications 3 à 7, **caractérisé en ce qu'**on disperse la matrice dans une phase huileuse qui est formée par les alcools de Guerbet à base d'alcools gras comprenant 6 à 18 atomes de carbone les esters d'acides gras linéaires en C₆-C₂₂ avec des alcools gras linéaires ou ramifiés en C₆-C₂₂ ; les esters d'acides carboxyliques ramifiés en C₆-C₁₃ avec des alcools gras linéaires ou ramifiés en C₆-C₂₂ ; les esters d'acides gras linéaires en C₆-C₂₂ avec des alcools gras ; les esters d'acides alkylhydroxycarboxyliques en C₁₈-C₃₈ avec des alcools gras linéaires ou ramifiés en C₆-C₂₂ ; les esters d'acides gras linéaires et/ou ramifiés avec des alcools polyvalents et/ou des alcools de Guerbet ; les triglycérides à base d'acides gras en C₆-C₁₀ ; les mélanges liquides de monoglycérides/diglycérides/triglycérides à base d'acides gras en C₆-C₁₈ ; les esters d'alcools gras en C₆-C₂₂ et/ou d'alcools de Guerbet avec des acides carboxyliques aromatiques ; les esters d'acides dicarboxyliques en C₂-C₁₂ avec des alcools linéaires ou ramifiés comprenant 1 à 22 atomes de carbone ou des polyols comprenant 2 à 10 atomes de carbone et 2 à 6 groupes hydroxyle ; les huiles végétales ; les alcools primaires ramifiés ; les cyclohexanes substitués ; les carbonates d'alcools gras linéaires et ramifiés en C₆-C₂₂ ; les carbonates de Guerbet à base d'alcools gras comprenant 6 à 18 atomes de carbone ; les esters d'acide benzoïque avec des alcools linéaires et/ou ramifiés en C₆-C₂₂ ; les dialkyléthers linéaires ou ramifiés, symétriques ou non symétriques comprenant 6 à 22 atomes de carbone par groupe alkyle ; les produits d'ouverture de cycle d'esters d'acides gras époxydés avec des polyols ; les huiles siliconées ainsi que les hydrocarbures aliphatiques ou naphténiques et les huiles minérales ainsi que leurs mélanges.

9. Procédé selon au moins l'une quelconque des revendications 3 à 8, **caractérisé en ce qu'**on utilise des polymères cationiques synthétiques qui sont choisis dans le groupe formé par les copolymères de sels de diallylammonium et d'acrylamides, de polymères quaternisés de vinylpyrrolidone/vinylimidazole, les produits de condensation et polyglycols et d'amines, les polyéthylèneimines, les polymères siliconés cationiques, les copolymères de l'acide adipique et de la diméthylaminohydroxypropyldiéthylènetriamine, les copolymères de l'acide acrylique avec du chlorure de diméthyldiallylammonium, les polyaminopolyamides ainsi que leurs polymères réticulés solubles dans l'eau, les produits de condensation de dihalogénoalkylènes et de bisdialkylamines ainsi que les polymères de sels d'ammonium quaternisés.

10. Utilisation de microcapsules selon la revendication 1 pour la préparation d'agents cosmétiques et/ou pharmaceutiques ou de préparations alimentaires.
